(19) Europäisches Patentamt
European Patent Office
Office européen des brevets

(11) Veröffentlichungsnummer : **0 070 424**
**B1**

(12) # EUROPÄISCHE PATENTSCHRIFT

(45) Veröffentlichungstag der Patentschrift : **11.12.85**

(51) Int. Cl.⁴ : **C 07 C102/08**, C 07 C103/50, C 07 D295/18

(21) Anmeldenummer : **82105751.0**

(22) Anmeldetag : **29.06.82**

(54) **Verfahren zur Herstellung von N,N'-disubstituierten 3-Aminopropanamiden.**

(30) Priorität : **20.07.81 DE 3128575**

(43) Veröffentlichungstag der Anmeldung : **26.01.83 Patentblatt 83/04**

(45) Bekanntmachung des Hinweises auf die Patenterteilung : **11.12.85 Patentblatt 85/50**

(84) Benannte Vertragsstaaten : **BE DE FR GB NL**

(56) Entgegenhaltungen :
**US-A- 4 031 138**
**US-A- 4 060 553**
**US-A- 4 201 854**

(73) Patentinhaber : **DEUTSCHE TEXACO AKTIENGESELLSCHAFT**
**Überseering 40**
**D-2000 Hamburg 60 (DE)**

(72) Erfinder : **Laping, Karlheinz, Dr.**
**Behringweg 1**
**D-4130 Moers 1 (DE)**
Erfinder : **Petersen, Olaf, Dr.**
**Comeniusstrasse 1**
**D-4050 Meerbusch 2 (DE)**
Erfinder : **Heinemann, Karl-Heinz, Dr.**
**Gartenstrasse 4**
**D-4133 Neukirchen-Vluyn (DE)**
Erfinder : **Humbert, Heiko, Dr.**
**Riepenhausenweg 5**
**D-2100 Hamburg 90 (DE)**

(74) Vertreter : **Schupfner, Gerhard et al**
**Patentanwälte Dipl.-Ing. Hans-Jürgen Müller**
**Dipl.-Chem.Dr.phil.nat. Gerhard Schupfner Dipl.-Ing.**
**Hans-Peter Gauger**
**Karlstrasse 5 D-2110 Buchholz in der Nordheide (DE)**

EP 0 070 424 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung von N,N'-disubstituierten 3-Aminopropanamiden der allgemeinen Formel

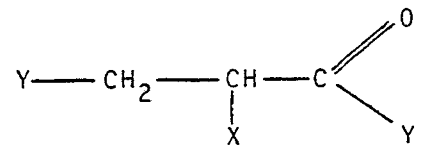

mit X = Wasserstoff oder Methyl
und Y =

NH—R—$R_1$, 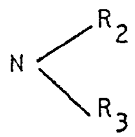 ,

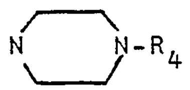 oder 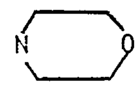 ,

worin

R einen gerad-, verzweigtkettigen oder cyclischen Alkylenrest mit 2 bis 8 C-Atomen oder Phenylen,

$R_1$ Wasserstoff, eine Dialkykamino- oder Alkoxygruppe, wobei der Alkylrest jeweils 1 bis 4 C-Atome enthält,

$R_2$, $R_3$, $R_4$ jeweils einen Alkylrest mit 1 bis 4 C-Atomen, wobei $R_2$ und $R_3$ auch zusammen einen Ring mit 5 oder 6 C-Atomen bilden können,

bedeuten.

Diese Verbindungen sind von technischem Interesse, da sie leicht in bekannter Weise durch Abspaltung der 3-ständigen Aminogruppe in N-substituierte Acrylamide bzw. Methacrylamide überführt werden können. N-substituierte Acrylamide bzw. Methacrylamide werden zum Beispiel als Monomere für die Herstellung von Homo- und Copolymerisaten in der Technik eingesetzt.

Es sind bereits Verfahren zur Herstellung von N,N'-disubstituierten 3-Aminopropanamiden bekannt.

So können 3-Aminopropanamide durch Umsetzung von Dialkylaminen mit Acrylsäure oder einem Acrylsäureester nach J. G. Erickson, J. Am. Chem. Soc. 74, 1952, 6281-6282 hergestellt werden.

Nach der US-Patentschrift 4 031 138 werden Diamine mit Acrylsäure oder einem Acrylsäurederivat zu disubstituierten 3-Aminopropanamiden umgesetzt.

Es bestand die Aufgabe, ein Verfahren bereitzustellen, das es gestattet, billigere und leichter verfügbare Ausgangsmaterialien als Acrylsäuren, deren Ester und andere Acrylsäurederivate einzusetzen.

Aus der US-Patentschrift 4 060 553 ist es bekannt, ungesättigte Nitrile mit einem Alkanolanion bei erhöhter Temperatur umzusetzen. Die Reaktionsbedingungen gemäß dieser Patentschrift konnten aber nicht auf die Umsetzung von Acryl- bzw. Methacrylnitril mit Aminen übertragen werden.

Es geht ferner aus der US-Patentschrift 4 201 854 hervor (siehe Beispiele 13 und 14), daß Acrylnitril mit einem Amin umgesetzt werden kann. Unter den Reaktionsbedingungen dieser Patentschrift lagert sich das Amin nur an die olefinische Doppelbindung an, ohne daß die Nitrilgruppe zur Umsetzung kommt.

In Lösung der gestellten Aufgabe betrifft die Erfindung ein Verfahren zur Herstellung der N,N'-disubstituierten 3-Aminopropanamiden der obigen allgemeinen Formel, das dadurch gekennzeichnet ist, daß Acryl- bzw. Methacrylnitril je Mol mit mindestens 2 Mol eines Amins der allgemeinen Formel

HY,

worin Y die oben angegebene Bedeutung besitzt, in Gegenwart von mindestens 1 Mol Wasser und ggf. an sich bekannten und für die Nitrilverseifung üblichen Katalysatoren bei 80 bis 220 °C und einem Druck von 5 bis 30 bar umgesetzt wird.

Es wurde gefunden, daß N,N'-disubstituierte 3-Aminopropanamide besonders leicht und in hoher Ausbeute durch das erfindungsgemäße Verfahren hergestellt werden können.

Als Beispiele für die eingesetzten Amine können genannt werden :

Primäre Amine : Ethylamin, n-Butylamin, Ethylhexylamin, Cyclohexylamin und insbesondere N,N-Dimethylpropandiamin, N,N,2,2-Tetramethylpropandiamin, 3-Methoxy-propylamin, N,N-Dimethyläthandiamin.

Sekundäre Amine : Dimethylamin, Di-butylamin, N-Methylpiperazin, Morpholin.

Aromatische Amine : Anilin, Toluidine, p-Methoxyanilin, p-Dimethylaminoanilin.

Acryl- bzw. Methacrylnitril wird im Überschuß, also mindestens mit der zwei- bis insbesondere etwa vierfachen molaren Menge an Amin, bezogen auf das Acrylnitril bzw. Methacrylnitril, in Gegenwart von Wasser, mindestens der ein- bis insbesondere der zwanzigfachen Menge Wasser und gegebenenfalls in Gegenwart eines Katalysators umgesetzt.

Außerhalb des Löslichkeitsbereiches des Nitril/Wasser-Gemisches läßt sich durch Variation der Menge des eingesetzten Amins ein homogenes Reaktionsgemisch erzeugen. Das Amin kann also gleichzeitig als Lösungsmittel wirken.

Die Umsetzung der drei Komponenten wird zwischen 80 und 220 °C und insbesondere 100 bis 180 °C bei etwa 5 bis 30 bar ggf. in Gegenwart von Katalysatoren durchgeführt. Dabei verläuft die Addition der Amine an das ungesättigte Nitril zu den 3-Aminopropannitrilen meist rasch. Die Geschwindigkeit des Verseifungsschrittes hängt vom eingesetzten Nitril und ggf. vom Katalysator ab. 3-Aminopropannitrile lassen sich schneller verseifen als 2-Methyl-3-aminopropannitrile. Durch die Verwendung von Katalysatoren lassen sich die Reaktionstemperaturen senken und die Reaktion dennoch wesentlich beschleunigen.

Bei Temperaturen unterhalb der angegebenen Grenze ist die Reaktion für einen technischen Prozeß zu langsam. Oberhalb 250 °C werden in zunehmendem Maße unerwünschte Nebenprodukte gebildet.

Führt man die Umsetzung über 100 °C durch, werden die sich intermediär bildenden Aminopropanamide sofort nach dem Entstehen durch vorhandenes Amin umamidiert.

Es finden ganz allgemein die in der Technik üblichen Nitril-Verseifungskatalysatoren Anwendung. So eignet sich besonders Kupfer sowohl als reines Metall, wie Raney-Kupfer, Ullmann-Kupfer, als auch in Verbindung mit Sauerstoff und anderen Metallen. Gut geeignet sind auch Kupfer-Katalysatoren, die weitere Metalle, wie Chrom, Molybdän, Vanadium, Mangan und Zink enthalten, sowie deren Mischoxide, wobei die angegebenen Metalle in den verschiedenen Oxidationsstufen auftreten können ; wie sie aus dem Handel bezogen oder nach einer Vielzahl von Methoden hergestellt werden können. Vor der Verwendung erfolgt eine übliche Wasserstoffreduktion, bei der das Oxid mit elementarem Wasserstoff bei geeigneten Temperaturen zur Erzielung der gewünschten Reduktion in Kontakt gebracht wird.

Die Katalysatoren können auch auf üblichen Trägern aufgebracht sein, wie auf Siliciumdioxid, Aluminiumoxid oder Aluminiumsilikaten.

Es muß darauf geachtet werden, daß die eingesetzten Verbindungen, so vor allem Wasser, weitgehend von Sauerstoff befreit sind. Dafür existieren eine Reihe technischer Verfahren.

Bei Temperaturen unterhalb der angegebenen Grenze ist die Reaktion für einen technischen Prozeß zu langsam. Oberhalb 250 °C werden in zunehmendem Maße unerwünschte Nebenprodukte gebildet.

Führt man die Umsetzung über 100 °C durch, werden die sich intermediär bildenden Aminopropanamide sofort nach dem Entstehen durch vorhandenes Amin umamidiert.

Führt man die Umsetzung unterhalb des angegebenen Temperaturgrenzbereiches durch, findet je nach Reaktionstemperatur während der Verseifung nur eine partielle Umamidierung (40 bis 80 %) statt. In diesem Falle wird nach Abtrennen des Katalysators zur Vervollständigung der Umamidierung kurze Zeit auf etwa 170 bis 200 °C erhitzt.

Es finden ganz allgemein die in der Technik üblichen Nitril-Verseifungskatalysatoren Anwendung. So eignet sich besonders Kupfer sowohl als reines Metall, wie Raney-Kupfer, Ullmann-Kupfer, als auch in Verbindung mit Sauerstoff und anderen Metallen. Gut geeignet sind auch Kupfer-Katalysatoren, die weitere Metalle, wie Chrom, Molybdän, Vanadium, Mangan und Zink enthalten, sowie deren Mischoxyde, weitere Metalle, wie Chrom, Molybdän, Vanadium, Mangan und Zink enthalten, sowie deren Mischoxide, dem Handel bezogen oder nach einer Vielzahl von Methoden hergestellt werden können. Vor der Verwendung erfolgt eine übliche Wasserstoffreduktion, bei der das Oxid mit elementarem Wasserstoff bei geeigneten Temperaturen zur Erzielung der gewünschten Reduktion in Kontakt gebracht wird.

Die Katalysatoren können auch auf üblichen Trägern aufgebracht sein, wie auf Siliciumdioxid, Aluminiumoxid oder Aluminiumsilikaten.

Es muß darauf geachtet werden, daß die eingesetzten Verbindungen, so vor allem Wasser, weitgehend von Sauerstoff befreit sind. Dafür existieren eine Reihe technischer Verfahren.

Ferner eignen sich auch Mangandioxid, Alkalihydroxide und Aluminiumsilikate natürlichen (Montmorillonite) und künstlichen Ursprungs als Katalysatoren der kombinierten Verseifungs- und Umamidierungsreaktion. Die Reaktivität nimmt im allgemeinen in der angegebenen Reihenfolge ab.

Die Umsetzung läßt sich sowohl als diskontinuierlicher Prozeß in einem Rührwerk oder auch als kontinuierlicher Prozeß in einem Reaktionsrohr mit Einlaß für die Reaktanten und Auslaß für die Produkte durchführen.

Arbeitet man in einem Rührwerk, werden nach der Umsetzung die dabei eingesetzten pulverförmigen Kupfer- oder Mangandioxid-Kontakte durch Filtration abgetrennt und, gegebenenfalls nach erneuter Aktivierung, wieder verwendet. Tablettierte Kontakte lassen sich in einem Reaktionsrohr fest anordnen.

Nach der Umsetzung werden Ammoniak, überschüssiges Wasser, Amin und nicht umgesetzetes Nitril destillativ entfernt.

Höher siedende Nitrile werden aufgrund der bei höheren Temperaturen einsetzenden pyrolytischen Zersetzung der hergestellten N,N'-disubstituierten 3-Aminopropanamide am besten durch eine schonende Dünnschicht- oder Kurzwegdestillation abgetrennt. Der Rückstand enthält die disubstituierten Propanamide in Reinheiten von mindestens 80 bis 95 % ; der rest sind jeweils Polymere.

Die Ausbeute beträgt je nach Verfahrensbedingungen 75 bis über 90 %.

Die disubstituierten Propanamide können je nach Siedepunkt durch Kolonnen-, Dünnschicht- oder auch Kurzwegdestillation in hoher Reinheit erhalten werden.

Das eben beschriebene Verfahren zur Herstellung von disubstituierten Propanamiden ist eine typische « Eintopfreaktion ». Alle drei Reaktionsschritte laufen gleichzeitig in einer Reaktionsmischung ab, so daß eine aufwendige Aufarbeitung von Zwischenprodukten entfällt. Darin liegt ein besonderer Vorteil des Verfahrens.

Die folgenden Beispiele erläutern die Erfindung :

## Beispiel 1

Ein Gemisch aus 265 g (5 Mol) Acrylnitril, 1 950 g (15 Mol) N,N,2,2-Tetramethylpropandiamin und 270 g (15 Mol) von Sauerstoff befreitem Wasser wurden mit 200 g Raney-Kupfer in einem 1 l-Autoklaven fünf Stunden auf 170 °C erhitzt. Danach wurde entspannt, der Kupferkatalysator abfiltriert, mit Wasser gespült, der entstandene Ammoniak, das überschüssige Amin und Wasser und nicht umgesetztes Aminopropannitril abgetrennt. Zur Abtrennung des Aminopropannitrils wurde eine Kurzwegdestillationsapparatur eingesetzt. Der viskose Rückstand enthielt ca. 90 % N,N'-Bis(2,2-dimethyl-3-dimethylaminopropyl)-3-aminopropanamid. Die Polymeranteile wurden durch Kurzwegdestillation abgetrennt, wodurch das disubstituierte Propanamid in einer Reinheit von über 98 % gewonnen wurde.

Die Ausbeute betrug 1 214 g. Diese sind 77,3 %, bezogen auf das eingesetzte Acrylnitril, bzw. etwa 92 %, wenn man berücksichtigt, daß ca. 15 % nicht umgesetztes Aminopropannitril zurückgewonnen wurde.

## Beispiel 2

Ein Gemisch aus 530 Teilen Acrylnitril, 3 900 Teilen N,N,2,2-Tetramethylpropandiamin und 900 g von Sauerstoff befreitem Wasser (Molverhältnis 1 : 3 : 5) wurde durch ein auf 150 °C erhitztes Rohr von 2 l Inhalt geleitet, das zur Hälfte mit Kupferchromitkontakt Harshaw 0203 T in reduzierter Form gefüllt war. Der Druck betrug dabei 20 bar, die Durchflußgeschwindigkeit 200 ml/h. Vom austretenden Reaktionsgemisch wurden zunächst der entstandene Ammoniak, das überschüssige Amin und das Wasser destillativ abgetrennt. In einer anschließenden Kurzwegdestillation wurde das entstandene N,N'-(2,2-dimethyl-3-dimethylaminopropyl)-3-aminopropanamid vom nicht umgesetzten Aminopropannitril befreit, das zusammen mit dem Amin und dem Wasser in den Rohrreaktor zurückgeführt wurde. Der Umsatz betrug pro Durchsatz etwa 50 %. Der viskose Rückstand bestand zu über 90 % aus Aminopropanamid, das durch eine Kurzwegdestillation von Polymeren abgetrennt und so in über 98 %iger Reinheit erhalten werden konnte.

## Beispiel 3

371 g (7 Mol) Acrylnitril, 2 730 g N,N,2,2-Tetramethylpropandiamin und 630 g Wasser wurden in einem Autoklaven dreißig Stunden auf 200 °C erhitzt. Danach wurde entspannt, der entstandene Ammoniak, das überschüssige Amin und Wasser und nicht umgesetztes Aminopropannitril destillativ entfernt. Zur Abtrennung des Aminopropannitrils wurde eine Kurzwegdestillationsapparatur eingesetzt.

Der viskose Rückstand (1 350 g) enthielt ca. 81 % N,N'-Bis(2,2-dimethyl-3-dimethylaminopropyl)-3-aminopropanamid (50 %). Die Polymeranteile wurden durch Kurzwegdestillation abgetrennt. Man erhielt ein disubstituiertes Propanamid von hoher Reinheit (über 98 %). Die Ausbeute betrug 1 092 g (49,7 % bzw. 75 %, wenn man berücksichtigt, daß etwa 25 % nicht umgesetztes Aminopropannitril zurückgewonnen werden konnten).

## Beispiele 4 bis 8

Ein 0,5 l-Autoklav wurde mit einem Drahtkorb versehen, der zur Aufnahme eines tablettierten Kupferkontaktes (Kupferoxid auf Silicatträger, 60/35 — Ruhrchemie) diente und außerdem Rühren und das Einführen von zwei am Autoklavendeckel vorhandenen Tauchungen gestattete. 200 g Kupferkatalysator Ruhrchemie 60/35 wurden eingefüllt und nach Vorschrift mit Wasserstoff reduziert.

Ein Gemisch aus 0,5 Mol Nitril, 1,5 Mol Amin und 2,5 Mol von Sauerstoff befreitem Wasser wurde je nach Amin drei bis fünf Stunden auf 100 bis 140 °C erhitzt. Der Katalysator wurde nun durch Herausheben des Drahtkorbes entfernt und der Autoklaveninhalt nochmals eine Stunde auf 140 bis 170 °C erhitzt. Nach beendeter Umsetzung wurde das erhaltene Amid durch Gaschromatographie quantitativ bestimmt. Die Ergebnisse sind in der Tabelle I wiedergegeben.

(Siehe Tabelle I Seite 5 f.)

Tabelle I

| Beispiel | Nitril-Komponente | Amin-Komponente | Bedingungen | Umsatz % | Produktbezeichnung |
|---|---|---|---|---|---|
| 4 | Acrylnitril | N,N-Dimethylpropandiamin | 3 h, 140 °C/<br>1 h, 170 °C | 88 | N,N'-Bis(3-dimethylaminopropyl)-3-aminopropanamid |
| 5 | Methacrylnitril | - " - | 3 h, 140 °C/<br>1 h, 170 °C | 82 | N,N'-Bis(3-dimethylaminopropyl)-3-amino-2-methylpropanamid |
| 6 | Acrylnitril | n-Butylamin | 3 h, 100 °C/<br>1 h, 140 °C | 87 | N,N'-Dibutyl-3-aminopropanamid |
| 7 | Acrylnitril | Dimethylamin | 2 h, 100 °C/<br>1 h, 140 °C | 85 | N,N,N',N'-Tetramethylpropanamid |
| 8 | Acrylnitril | N-Methylpiperazin | 5 h, 140 °C/<br>1 h, 170 °C | 46 | N,N'-Bis(N"-methylpiperazyl)-3-aminopropanamid |

Der entstandene Ammoniak, das überschüssige Amin, nicht umgesetztes Aminopropannitril und Wasser wurden über eine kurze Kolonne abdestilliert. Das im Rückstand verbleibende disubstituierte Propanamid wurde je nach Siedepunkt und Pyrolyseneigung über eine Kolonne oder einen Kurzwegverdampfer destilliert. Dadurch wurden sowohl noch enthaltene Reste leichtsiedender Verbindungen als auch Polymere abgetrennt. Die reinen disubstituierten Aminopropanamide wurden durch IR- und NMR-Spektren identifiziert.

**Patentansprüche**

1. Verfahren zur Herstellung von N,N'-disubstituierten 3-Aminopropanamiden der allgemeinen Formel

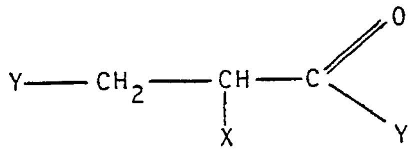

mit X = Wasserstoff oder Methyl
und Y =

$NH—R—R_1$, 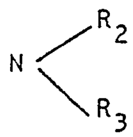

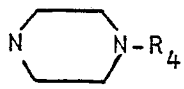 oder 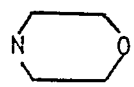

worin

R einen gerad-, verzweigtkettigen oder cyclischen Alkylenrest mit 2 bis 8 C-Atomen oder Phenylen,

$R_1$ Wasserstoff, eine Dialkylamino- oder Alkoxygruppe, wobei der Alkylrest jeweils 1 bis 4 C-Atome enthält,

$R_2$, $R_3$, $R_4$ jeweils einen Alkylrest mit 1 bis 4 C-Atomen, wobei $R_2$ und $R_3$ auch zusammen einen Ring mit 5 oder 6 C-Atomen bilden können,

bedeuten,

dadurch gekennzeichnet, daß man Acryl- bzw. Methacrylnitril je Mol mit mindestens 2 Mol eines Amins der allgemeinen Formel

HY,

worin Y die oben angegebene Bedeutung besitzt, in Gegenwart von mindestens 1 Mol Wasser und gegebenenfalls an sich bekannten und für die Nitrilverseifung üblichen Katalysatoren bei 80 bis 220 °C und einem Druck von 5 bis 30 bar umsetzt.

2. Verfahren nach Anspruch 1, dadurch gekennzeichnet, daß man die Umsetzung bei 100 bis 180 °C durchführt.

3. Verfahren nach Anspruch 1 oder 2, dadurch gekennzeichnet, daß man die Umsetzung im Autoklaven bei autogenem Druck durchführt.

4. Verfahren nach einem der vorhergehenden Ansprüche, dadurch gekennzeichnet, daß man 2 bis 7 Mol Amin je Mol Nitril einsetzt.

5. Verfahren nach einem der vorhergehenden Ansprüche, dadurch gekennzeichnet, daß man Wasser im Überschuß bis zur 20-fach molaren Menge einsetzt.

6. Verfahren nach einem der vorhergehenden Ansprüche, dadurch gekennzeichnet, daß man als Katalysator einen Kupferkatalysator als Metall, Oxid oder Chromit mit oder ohne Träger einsetzt.

**Claims**

1. Process for producing N,N'-disubstituted 3-aminopropanamides of the general formula

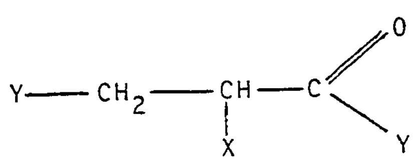

wherein X is hydrogen or methyl
and Y is

NH—R—$R_1$, 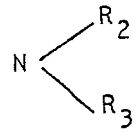

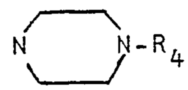 or 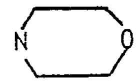

wherein

R is a linear, branched, or cyclic alkylene residue with 2 to 8 carbon atoms, or phenylene,

$R_1$ is hydrogen, a dialkylamino or alkoxy group, the alkyl residue having 1 to 4 carbon atoms each,

$R_2$, $R_3$, $R_4$ each are an alkyl residue with 1 to 4 carbon atoms, where $R_2$ and $R_3$ may also form together a ring with 5 or 6 carbon atoms,

characterized by reacting one mole of acryl or methacrylnitrile each with at least 2 moles of an amine of the general formula

HY,

wherein Y has the abovementioned significance, in the presence of at least 1 mole of water and, optionally, with catalysts known per se and usually employed for the saponification of nitrile, at 80 to 220 °C and a pressure of 5 to 30 bar.

2. Process according to claim 1, characterized by performing the reaction at 100 to 180 °C.

3. Process according to claim 1 or 2, characterized by performing the reaction in an autoclave under autogenous pressure.

4. Process according to any one of the preceding claims, characterized by using 2 to 7 moles of amine per mole of nitrile.

5. Process according to any one of the preceding claims, characterized by using water at an excess of up to the twentyfold molar amount.

6. Process according to any one of the preceding claims, characterized by using as catalyst a copper catalyst as a metal, oxide, or chromite with or without carrier.

**Revendications**

1. Procédé de production de 3-aminopropanamides N,N'-disubstitués de formule générale

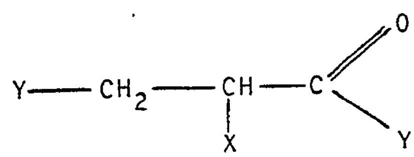

dans laquelle X = hydrogène ou méthyle
et Y =

NH—R—$R_1$, 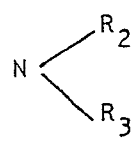

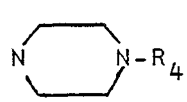 ou 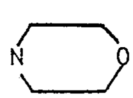

R représentant un reste alkylène à chaîne droite, à chaîne ramifiée ou cyclique ayant 2 à 8 atomes de carbone ou le reste phénylène,

$R_1$ représentant l'hydrogène, un groupe dialkylamino ou alkoxy, le reste alkyle ayant dans chaque cas 1 à 4 atomes de carbone,

$R_2$, $R_3$, $R_4$ représentant chacun un reste alkyle ayant 1 à 4 atomes de carbone, $R_2$ et $R_3$ pouvant aussi former ensemble un noyau de 5 ou 6 atomes de carbone,

caractérisé en ce qu'on fait réagir l'acrylonitrile ou le méthacrylonitrile avec, pour chaque mole, au moins deux moles d'une amine de formule générale

HY,

dans laquelle Y a la définition indiquée ci-dessus, en présence au moins d'une mole d'eau et le cas échéant sur des catalyseurs connus et classiques pour la saponification d'un nitrile, à une température de 80 à 220 °C et sous une pression de 5 à 30 bars.

2. Procédé suivant la revendication 1, caractérisé en ce qu'on conduit la réaction à 100-180 °C.

3. Procédé suivant la revendication 1 ou 2, caractérisé en ce qu'on conduit la réaction dans l'autoclave à la pression autogène.

4. Procédé suivant l'une des revendications précédentes, caractérisé en ce qu'on utilise 2 à 7 moles d'amine par mole de nitrile.

5. Procédé suivant l'une des revendications précédentes, caractérisé en ce qu'on utilise de l'eau en excès allant jusqu'à 20 fois la quantité molaire.

6. Procédé suivant l'une des revendications précédentes, caractérisé en ce qu'on utilise comme catalyseur un catalyseur au cuivre sous la forme de métal, d'oxyde ou de chromite avec ou sans support.